# EUROPÄISCHE PATENTSCHRIFT

(11) **EP 0 353 619 B1**
(45) Veröffentlichungstag und Bekanntmachung des Hinweises auf die Patenterteilung: **12.12.2001**
(21) Anmeldenummer: 89113803.4
(22) Anmeldetag: 26.07.1989
(51) Int. Cl.: A61B 5/00, G01N 21/31

(54) **Verfahren zur Bestimmung von lokalen Farbstoff-Konzentrationen in tierischen und menschlichen Geweben**
Process for determination of local dye-concentrations in animal and human tissue
Méthode pour déterminer les concentrations locales de la matière colorante dans les tissus d'animaux et d'humains

(30) Priorität: 26.07.1988 DE 3825352
(43) Veröffentlichungstag der Anmeldung: 07.02.1990
(73) Patentinhaber: Kessler, Manfred, Prof. Dr. med., D-91056 Erlangen (DE)
(72) Erfinder: Kessler, Manfred, Prof. Dr. med., D-91056 Erlangen (DE); Frank, Klaus, Dr., D-91056 Erlangen (DE)
(74) Vertreter: Hofmann, Gerhard, Dipl.-Ing. Patentassessor

(56) Entgegenhaltungen:
- EP-A- 0 046 601
- DE-A- 3 008 651
- PHYSICS IN MEDICINE & BIOLOGY Band 33, Nr. 6, Juni 1988, Seiten 711-722, Bristol, GB; J.W. FEATHER et al. "A portable reflectometer for the rapid quantification of cutaneous haemoglobin and melanin"

## Beschreibung

Die Erfindung betrifft ein Verfahren zur Bestimmung von lokalen Farbstoff-Konzentrationen in tierischen und menschlichen Geweben, bei dem Licht unterschiedlicher Wellenlängen in einen Teilbereich des Gewebes eingestrahlt wird, zumindest ein Teil des rückgestreuten Lichtes aufgefangen und die Remission in Abhängigkeit von der Wellenlänge bestimmt wird und die Konzentration von Farbstoffen aus dem spektralen Remissionsgrad bestimmt wird, wobei in einem Schritt Strahlung aus einem ersten Wellenlängenbereich (I) in dem der Einfluß des Hämoglobins (Hb) auf die Remission gering ist, eingestrahlt und die Remission (MI0) im ersten Wellenlängenbereich (I) bestimmt wird und wobei in einem weiteren Schritt Licht aus einem zweiten Wellenlängenbereich (II), in dem die Remission vom Einfluß des Hämoglobins dominiert wird, in den gleichen Teilbereich des Gewebes eingestrahlt wird und die Remission (MII0) im zweiten Wellenlängenbereich (II) bestimmt wird und wobei die Daten über die Remission abgespeichert werden.

Ein solches Verfahren ist z. B. aus der Dissertation "Bestimmung von HämoglobinOxygenierung und relativer Hämoglobin-Konzentration in biologischen Systemen durch Auswertung von Remissionsspektren mit Hilfe der Kubelka-Munk-Theorie" von Wolfgang Dümmler, Erlangen 1988, bekannt.

Unter "lokaler" Konzentration soll hier insbesondere und beispielsweise der intrakapilläre Bereich verstanden werden.

Unter "Farbstoffen" sollen einerseits gewebeeigene Farbstoffe (Pigmente), insbesondere Hämoglobin, aber auch Zytochrome, aber auch zugeführte Farbstoffe, bei denen dann bspw. die Auswaschkinetik untersucht wird, verstanden werden.

"Licht unterschiedlicher Wellenlängen" ist in der Regel das Mischlicht einer Lampe (z. B. einer Xenon-Hochdrucklampe), kann aber z. B auch das Licht einer abstimmbaren Laserlichtquelle sein. Das Licht wird meist erst nach der Remission spektral zerlegt und die Intensität in Abhängigkeit von der Wellenlänge ausgewertet, wobei die spektral unterschiedlichen Anfangsintensitäten rechnerisch berücksichtigt werden.

Unter einem "Teilbereich" wird ein Bereich mit verhältnismäßig kleiner Oberfläche, typischerweise im Bereich von 50 - 500 µm Durchmesser, verstanden. Die Tiefenerstreckung im Gewebe hängt von zahlreichen Faktoren ab und liegt (Abfall auf 1/e) in der Größenordnung von - 150 µm. Wie weiter unten noch ausgeführt wird, ist aber das Gewebevolumen, aus dem die Remission erhalten wird, sowohl gewebespezifisch aus auch gerätespezifisch und ferner noch abhängig von der Hämoglobin-Konzentration.

Unter "Grundremission" wird hier die Remission des hämoglobinfreien Gewebes verstanden, wie sie bspw. bei einer hämoglobinfreien Perfusion des Gewebes festgestellt werden kann.

Unter "gewebespezifisch" werden die Besonderheiten verstanden, die aus der speziellen Art des Gewebes (z. B. Rattenleber oder menschliche Haut) resultieren. Mit "gewebepersonenspezifisch" werden solche Werte und Kurven bezeichnet, in die die konkrete Messung zumindest einer der beiden Remissionskurven schon eingegangen ist, und sei es nur durch die durch die Messung beeinflußte Auswahl aus einer vorab ermittelten Kurvenschar.

Die vorliegende Erfindung geht daher von einem Stand der Technik aus, wie er durch die vorgenannte Dissertation gegeben ist. Dort ist auch eine Vorrichtung zur Messung der Spektren von Hämoglobinkonzentrationen in Geweben erläutert, wobei diese Spektren in einem Rechner gespeichert werden können. Wie in der Dissertation weiter ausgeführt wird, stößt jedoch beispielsweise die Absolutmessung der Hämoglobinkonzentration mit einer derartigen Anordnung auf beträchtliche Schwierigkeiten. Die Erfindung schafft daher ein Verfahren gemäß Anspruch 1, das ermöglicht, die Hämoglobinkonzentration wesentlich genauer zu bestimmen.

Die Erfindung macht sich für die Bestimmung des Absolutwerts der Hämoglobin-konzentration zu nutze, daß im ersten Wellenlängenbereich des eingestrahlten Lichts, das Hämoglobin ein Fenster hat, so daß die Grundremission verhältnismäßig ungestört erkennbar wird, der Einfluß der Grundremission auch im zweiten Wellenlängenbereich, wo sie vom Hämoglobineinfluß in aller Regel völlig überlagert wird, genauer, abgeschätzt und entsprechend eliminiert werden kann. Die so gewonnenen, genaueren Werte können in weiteren Verfahrensschritten noch verfeinert werden.

Es wird vorab eine Schar gewebeartsspezifischer Standard-Grundremissionskurven an Gewebeproben der gleichen Gewebeart gewonnen, und wird die gemessene Remissionskurve im ersten Wellenlängenbereich dem nächstpassenden Ast im ersten Wellenlängenbereich aus der Schar der Standard-Grundremissionskurven zugeordnet und der zugehörige Ast dieser Standard-Grundremissionskurve im zweiten Wellenlängenbereich als gewebepersonenspezifische Standard-Grundremissionskurve ausgewählt.

Unter Standard-Grundremissionskurven werden Grundremissionskurven verstanden, die "vorab" an einer großen Zahl von Geweben der zu messenden Gewebeart durch Perfusion, z. B. durch hämoglobinfreie Perfusion gemessen und gespeichert wurden. In dieser Ausführungsform der Erfindung werden Kurven ermittelt, die als Kurvenschar bei einer Wellenlänge einen großen Bereich von Remissionen überdecken, ohne daß sich die einzelnen Kurven schneiden. Aus der Schar dieser Kurven wird nun die Kurve ausgewählt, die der gemessenen, zu untersuchenden Kurve im ersten Wellenlängenbereich am nächsten kommt und der im zweiten Wellenlängenbereich liegende Ast dieser ausgewählten (gewebeartspezifischen) Standard-Grundremissionskurve wird als gewebepersonenspezifische Standard-Grundremissionskurve definiert.

Dies hat insbesondere den Vorteil, daß nach der Erstellung einer solchen Kurvenschar auf einfache Weise aus der im ersten Wellenlängenbereich gemessenen Kurve auf den noch unbekannten Verlauf im zweiten Wellenlängenbereich geschlossen werden kann.

Dabei erfolgt die Zuordnung einer Standard-Grundremissionskurve aus der Schar der vorab gewonnenen Standard-Grundremissionskurven im ersten Wellenlängenbereich zu der gemessenen Remissionskurve derart, daß die Standard-Grundremissionskurve mit dem Wert an einer vorbestimmten isobestschen Wellenlänge im ersten Wellenlängenbereich, der gleich oder nächst dem gemessenen Remissionswert an dieser isosbestschen Wellenlänge ist, ausgewählt wird, und als Wert zur Ermittlung der Hämoglobinkonzentration der Wert der ausgewählten Standard-Grundremissionskurve an einer vorbestimmten isosbestschen Wellenlänge im zweiten Wellenlängenbereich benutzt wird.

Die Werte an den isosbestschen Wellenlängen werden genommen, weil sich dort kein zusätzlicher Fehler durch die zunächst noch unbekannte Oxygenierung des Hämoglobins ergibt. Diese Werte reichen zu dem genannten Zweck aber auch aus, weil schon der um die Grundremission bereinigte Remissionswert der Remissionskurve an einer isosbestschen Wellenlänge ausreicht, um aus dem Remissionswert mit einem entsprechend geeichten Gerät die Konzentration zu bestimmen.

Der Remissionswert, der den Schluß auf die Hämoglobinkonzentration erlaubt, wird in aller Regel durch Subtraktion des gewonnenen Wertes an der isobestschen Wellenlänge im zweiten Wellenlängenbereich vom gemessenen Remissionswert an dieser Wellenlänge ermittelt.

Gemäß der Erfindung wird zur Bestimmung der Oxygenierung des Hämoglobins vorab anhand einer Vielzahl von Messungen an der gleichen Gewebeart, mit Hämoglobin unterschiedlicher Konzentration und unterschiedlicher Oxygenierung, eine zweidimensionale Schar von Vergleichskurven erstellt und tabellarisch oder in einer Matrix angeordnet und gespeichert. Die Vergleichskurven mit Hämoglobinkonzentrationen in der Umgebung der ermittelten Konzentrationen werden über den ganzen Oxygenierungsbereich durchsucht, wobei die bestpassende der Vergleichskurven einen angenommenen Wert für die Oxygenierung und einen verbesserten Wert für die Konzentration liefert.

Auf diese Weise lassen sich wiederum einfach und schnell mit Hilfe der gemessenen Remissionen und vorbekannter Standardwerte zuverlässige Werte für die Hämoglobinkonzentration gewinnen.

Bevorzugt werden die auf die eben geschilderte Weise gewonnenen Werte für die Konzentration und die Oxygenierung bei dem geschilderten Schritt zur Gewinnung einer verbesserten gewebepersonenspezifischen Grundremissionskurve verwendet.

Bevorzugt wird in einer Weiterführung des Verfahrens anhand des für die Hämoglobinkonzentration gewonnenen Wertes die gemessene Kurve im ersten Wellenlängenbereich korrigiert, wodurch eine weitere, verbesserte Näherung für die gewebepersonenspezifische Grundremission im ersten Wellenlängenbereich gewonnen wird.

Die gemessene Remissionskurve im ersten Wellenlängenbereich, die ja noch den, allerdings dort geringen Einfluß der Hämoglobinkonzentration enthielt, war im obigen Verfahren eine "nullte Näherung" einer gewebepersonenspezifischen Grundremissionskurve im ersten Wellenlängenbereich. Diese nullte Näherung läßt sich nun verbessern, indem die (ihrerseits aus obigen Verfahrensschritten in ersten Näherung bekannte) Hämoglobinkonzentration aus der Kurve eliminiert wird. Die so erhaltene weitere Näherung wird vorteilhaft in die oben geschilderten Verfahrensschritte anstelle der gemessenen Remissionskurve eingebracht.

Es werden also besonders bevorzugt mit der verbesserten Kurve anstelle der gemessenen Remissionskurve Näherungsschritte durchgeführt, wodurch ein besserer Näherungswert für die Hämoglobinkonzentration und eine weiter verbesserte Kurve als gewebepersonenspezifische Grundremission im ersten Wellenlängenbereich erhalten werden.

Bevorzugt werden die obigen Schritte n-fach, unter Zugrundelegung der jeweils verbesserten Werte bzw. Kurven, wiederholt.

Als breiterer erster Wellenlängenbereich wird der Bereich von 630 nm bis 1000 nm bevorzugt.

Als engerer erster Wellenlängenbereich wird der Wellenlängenbereich von 750 nm bis 850 nm bevorzugt. In diesen Bereichen hat das Hämoglobin ein Fenster, sein Einfluß auf die Geweberemission ist also gering.

Als breiterer zweiter Wellenlängenbereich wird der Wellenlängenbereich von 500 nm bis 620 nm bevorzugt.

Als engerer zweiter Wellenlängenbereich wird der Bereich von 550 nm bis 570 nm bevorzugt. In den letzteren beiden Bereichen ist der Einfluß des Hämoglobins auf die Geweberemission groß.

Besonders bevorzugt wird die gemessene Remissionskurve aus dem zweiten Wellenlängenbereich auf die nächstpassende Kurve aus der oben erwähnten, zur Bestimmung der Oxygenierung dienenden, zweidimensionalen Schar normiert, die Differenz zwischen den beiden Kurven wellenlängenabhängig aufgetragen und als Maß der Verzerrung zur Ermittlung der Eindringtiefe des eingestrahlten Lichtes, d. h. des durch das Licht erfaßten Volumens V, benutzt.

Es hat sich herausgestellt, daß diese Verzerrung als Maß der Eindringtiefe benutzt werden kann. Entsprechende vorab ermittelte Verzerrungskurven werden wiederum gespeichert, ihnen wird das entsprechende Volumen zugeordnet, und dann wird die durch den Vergleich erhaltene Verzerrungskurve aus den gespeicherten Verzerrungskurven zugeordnet und so das Volumen bestimmt.

Zu den obigen Messungen wird ein in Erlangen entwickeltes MikroLichtleiterspektrophotometer verwendet. Dies wird weiter unten noch näher erläutert. Dabei weist die darin verwendete Interferenz-Verlauffilterscheibe bevorzugt sowohl den ersten Wellenlängenbereich als auch den zweiten Wellenlängenbereich auf. Dadurch können die gemessenen Remissionskurven des ersten und des zweiten Wellenlängenbereichs in einem Umlauf der Scheibe erhalten werden.

Bevorzugt weist das Mikrolichtleiterspektrophotometer Mittel zur absoluten Eichung des illuminierenden und detektierenden Systems auf. Die Absoluteichung ist besonders wichtig, da die Vorabmessungen, die ohnehin mit dem gleichen Gerät erfolgen sollten bzw. gerätespezifisch umgerechnet werden müssen, unter zu denen der konkreten Messung vergleichbaren, definierten Bedingungen stattfinden müssen. Solche Mittel sind insbesondere ein Weißstandard und eine Normallichtquelle, die unten noch erläutert werden.

Wie weiter unten bei der Beschreibung noch dargestellt werden wird, kann die Vorrichtung eine Interferenz-Verlaufsfilterscheibe enthalten, die die entsprechenden Wellenlängenbereiche enthält, vorzugsweise in einer einzigen Scheibe vereinigt. Eine Auswahl bestimmter Wellenlängenbereiche kann auch dadurch getroffen werden, daß, da die Auswert-Vorrichtung die jeweilige Stellung der Interferenz-Verlaufsfilterscheibe berücksichtigt, von der Auswert-Vorrichtung nur die gewünschten Wellenlängen (bzw. die gemessenen Werte an diesen Wellenlängen) weiterverarbeitet werden.

Die Erfindung ermöglicht auch die Ermittlung von Größenveränderungen an Gewebepartikeln. Die Beobachtung solcher Veränderungen, z. B. der Größenänderung von Mitochondrien, ist von besonderer praktischer Bedeutung, da sie z. B. ermöglicht, ein Gehirnödem frühzeitig zuerkennen.

Nachfolgend wird die Erfindung anhand bevorzugter Ausführungsbeispiele unter Bezugnahme auf die beigefügten Zeichnungen, noch näher erläutert. Es zeigen:
- Fig. 1:: Eine typische, an Rattenleber gemessene, schon auf Weißlichteinstrahlung korrigierte, Remissionskurve MIO im Wellenlängenbereich I, sowie die gleiche Kurve nach Berücksichtigung der Hämoglobinkonzentration in erster Näherung, GI1; sowie im Wellenlängenbereich II eine gemessene Remissionskurve MIIO, praktisch gleichzeitig mit der Kurve MIO gemessen, sowie die zur Kurve MIO gehörende Standard-Grundremissionskurve SIO;
- Fig. 2:: Der Kurve MIO entsprechende Kurven, bei hoher (M'I0ₕ) und niedriger (M'I0ₙ) Hämoglobinoxygenierung; sowie eine zugehörige Grundremissionskurve;
- Fig. 3:: Auf der perfundierten Rattenleber gemessene Standard-Grundremissionskurven bei hoher (SIIₕ) bzw. niedriger (SIIₙ) Oxydation der Atmungsfermente;
- Fig. 4:: Eine Wertematrix (Auszug) für die Zuordnung gemessener Remissionen bei der Wellenlänge = 815 nm (isosbestsche Wellenlänge des Hämoglobins im Wellenlängenbereich I) zu Standard-Remissionswerten bei = 586 nm (einer isosbestschen Wellenlänge in II) im Wellenlängenbereich II;
- Fig. 5:: Schematisch eine Wertematrix, deren Felder Standard-Hämoglobinspektren der Gewebeart des zu untersuchenden Gewebes enthalten, wobei die Zeilen Kurven mit jeweils unterschiedlicher Konzentration bei gleichem Oxygenierungsgrad und die Spalten Kurven mit jeweils gleicher Konzentration bei unterschiedlichem Oxygenierungsgrad enthalten;
- Fig. 6:: Schematisch die Einstrahlung mittels Lichtleiter und die Detektion mittels eines weiteren Lichtleiters und die dadurch erreichten Volumina im Gewebe;
- Fig. 7:: Schematisch drei Kurven, die zur Ermittlung der Verzerrung zweier gemessener Kurven dienen;
- Fig. 8:: Die sich aus den Kurven der Fig. 7 ergebenden Differenz- oder Verzerrungskurven;
- Fig. 9:: Eine Anordnung aus mehreren beleuchteten Lichtleitern und mehreren aufnehmenden Lichtleitern, zur Erstellung einer Topographie von Farbstoffverteilungen;
- Fig. 10:: Schematisch das in Erlangen entwickelte Mikrolichtleiter-Spektrophotometer, das zu den Messungen verwendet wird;
- Fig. 11:: Eine Anordnung zur Eichung des Gerätes aus Fig. 10;
- Fig. 12:: Kurven, die Eichkurven und eine gemessene Kurve zeigen.
- Fig. 13:: Eine unter Berücksichtigung der Eichkurve korrigierte Meßkurve.

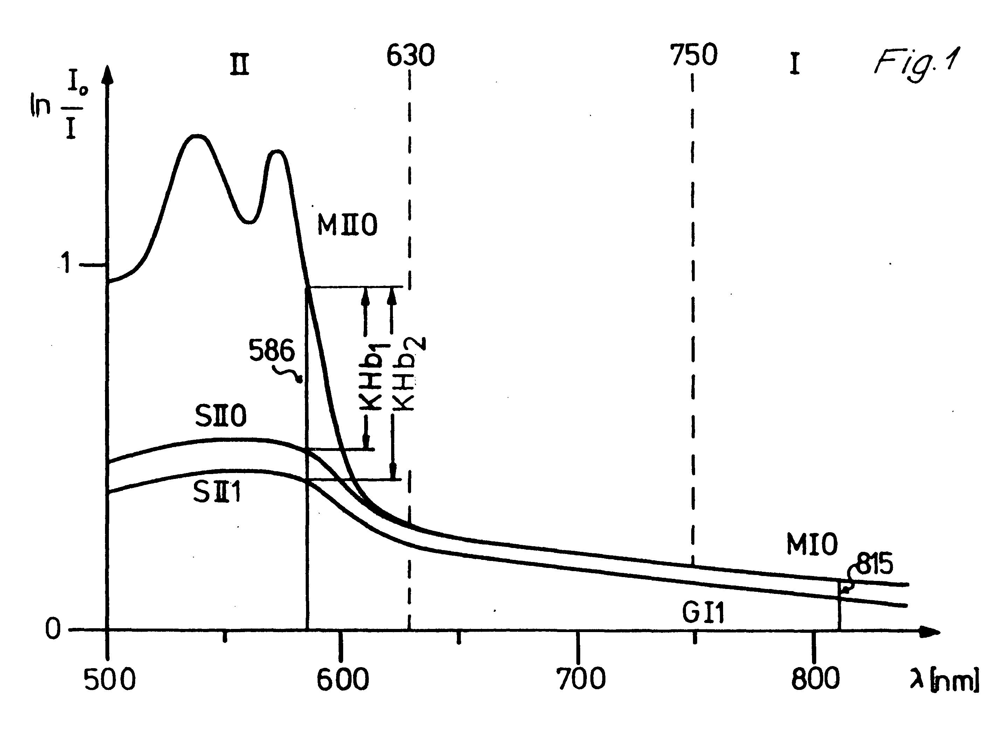

Figur 1 zeigt rechts eine erste gemessene Kurve MIO (M soll gemessen, I soll auf den Wellenlängenbereich I (hier: 750 - 850 nm) hinweisen, und die Ziffer O gibt an, daß es sich um eine Kurve handelt, die als die nullte Näherung der Grundremissionskurve betrachtet werden kann). Als Maß für die Remission wir hier In I₀/I verwendet. Die Kurve wurde an Rattenleber mit dem MikrolichtleiterSpektrophotometer gemessen, das u. a. in der Dissertation "Optische Streuung an biologischen Partikeln und Zellen", Erlangen 1985, des einen der Erfinder, Frank, näher erläutert ist, aber auch weiter unten noch beschrieben wird.

Die Kurve weist nur einen geringen Einfluß des Hämoglobins auf, da Hämoglobin im Bereich I ein Fenster hat.

Unter "Grundremission" wird hier die Remission des hämoglobinfreien Gewebes verstanden, wie sie bspw. bei einer hämoglobinfreien Perfusion des Gewebes festgestellt werden kann. Diese Grundremission ist noch gewebeartspezifisch, sie hängt vom Redoxzustand der verbleibenden Zellpigmente und evtl. zugefügter Farbstoffe ab und ist auch noch, wenn auch nur in geringem Ausmaß, gewebepersonenspezifisch. Deshalb ist es erforderlich, die konkrete "wahre" Grundremission möglichst genau zu bestimmen, um die Konzentration, Oxygenierung und den Redoxzustand verschiedener Pigmente/Farbstoffe ermitteln zu können.

Die (systematisch betrachtet in einem Verfahrensschritt 1.1) gemessene Kurve MIO stellt im Verfahren eine (nullte) Näherung dar, weil sie noch von dem, im gewählten Wellenlängenbereich I von 750 bis 850 nm allerdings geringen, Einfluß der Hämoglobinkonzentration (KHb) sowie der Oxygenierung des Hämoglobins (HbO₂/Hb) abhängig ist.
In einem weiteren Schritt (2.) wird zu der gemessenen Grundremissionskurve MIO eine zugehörige Standard-Remissionskurve im Wellenlängenbereich II ausgewählt.

Zur Ermittlung der daher so genannten "Standard"-Grundremissionskurven wird (sozusagen in einem 0. Verfahrensschritt) an einer großen Zahl (ca. 100) hämoglobinfrei perfundierter in vivo- und in vitro-Proben ermittelt, welche Remissionskurven sich im Bereich II bei bestimmten Remissionen im Bereich I ergeben. Es wird also eine große Menge von Wertescharen ermittelt, wobei die Wertescharen Werte einer Kurve bei den verschiedenen Wellenlängen aus den Wellenlängenbereichen I und II enthalten.

In der konkreten Tabelle (Fig. 4) ist einer Folge (je ein Wert aus den jeweils eine Kurve bildenden Wertescharen) von Remissionen bei einer (hier "der") isosbestschen Wellenlänge des Hämoglobins im Wellenlängenbereich I eine Folge an einem isosbestschen Punkt des Hämoglobins im Bereich II eindeutig zugeordnet (derart, daß die Werte des Wertepaares auf der selben Standard-Remissionskurve liegen). Die Tabelle stellt also einen engsten Ausschnitt aus den jeweiligen Kurven, bei den angegebenen Wellenlängen, dar.

Die isosbestsche Wellenlänge des Hämoglobins (815) nm) wurde im Wellenlängenbereich I ausgewählt, da der Einfluß der Oxygenierung des Hämoglobins auf die Remission im Wellenlängenbereich I größer ist als der Einfluß der Oxydation der Zytochrome, welcher Einfluß erst bei einem Sauerstoff-Partialdruck von weniger als 5 Torr sich merklich ändert. Durch die Wahl der isosbestschen Wellenlänge wird man von der in diesem Schritt noch unbekannten Oxygenierung des Hämoglobins unabhängig. Der Fehler der nullten Näherung, der gemessenen Kurve MIO, hängt dort nur noch von der Konzentration des Hämoglobins ab.

Zur Zuordnung einer Standard-Grundremissionskurve (SII0) nullten Näherung im Wellenlängenbereich II wird also im Wellenlängenbereich I der Wert der Folge an der isosbestschen Wellenlänge gewählt, der gleich dem oder nächstliegend zu dem gemessenen Wert an der isosbestschen Wellänge auf der Kurve MI0 ist und diesem mittels der Tabelle 2 der zugehörige Wert der Folge im Wellenlängenbereich II, und damit auch der ganze Ast der entsprechenden Standard-Grundremissionskurve in II, zugeordnet.

Im Bereich II wird ebenfalls eine isosbestsche Wellenlänge (im Ausführungsbeispiel konkret 586 nm) gewählt, um von der noch unbekannten Oxygenierung unabhängig zu sein.

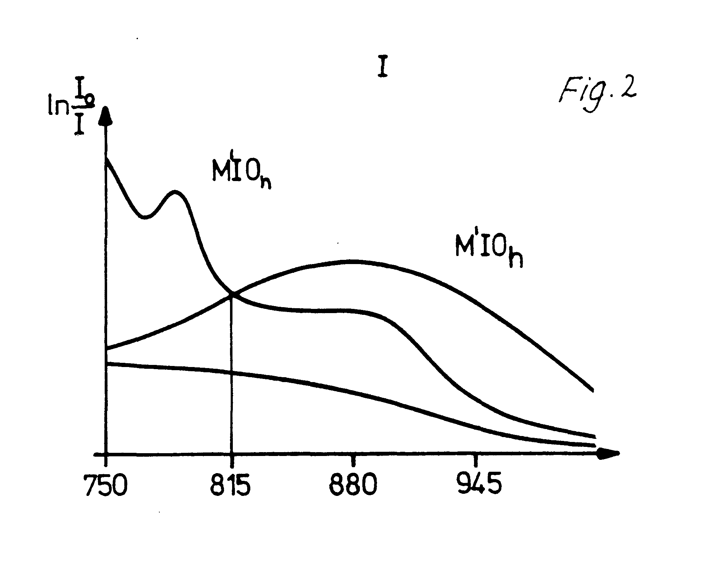

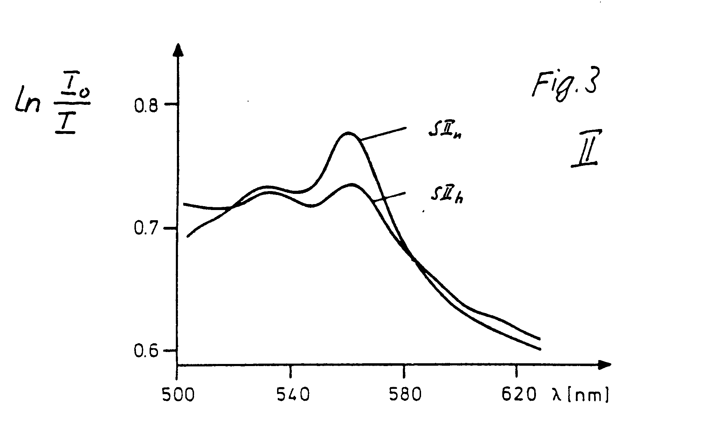

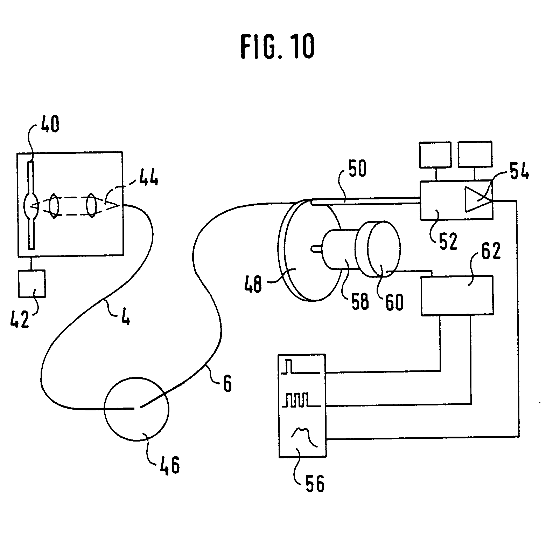

Fig. 1 zeigt in der linken Hälfte eine im gleichen Durchgang einer Interferenz-Verlaufsfilterscheibe (s. u. Fig. 10) gewonnene, (systematisch betrachtet in einem Schritt 1.2.) gemessene Remissionskurve an der gleichen Gewebestelle. Diese wird als gemessene Kurve im Wellenlängenbereich II und nullte Näherung, als MII0 bezeichnet. Ferner ist in Fig. 1 links die zum im ersten Wellenlängenbereich I ermittelten Meßwert gehörende Standard-Grundremissionskurve SII0 eingezeichnet, die ebenfalls, wie oben geschildert, als Tabelle vorliegt (ein Ausschnitt ist in Fig. 2 gezeigt), oder aus dem Vergleich der gemittelten Standard-Grundremissionskurve im Bereich I mit der Kurve MI0, wie oben alternativ geschildert, ermittelt wird. An der isosbestschen Wellenlänge wird aus der gemessenen Gesamtremission (Kurve MII0), die sich aus der Grundremission und der hämoglobinabhängigen Remission zusammensetzt, durch Subtraktion des Wertes der Standard-Grundremissionskurve SII0 an der konkreten isosbestschen Wellenlänge (586 nm) von dem Wert der gemessenen Remissionskurve MII0 die Hämoglobin-Remission und damit ein Maß für die Hämoglobin-Konzentration in erster Näherung, KHb1, (in einem 3. Schritt) gewonnen. Damit wird ein erster Näherungswert, KHb1, für die Konzentration des Hämoglobins erhalten.

Dieser Konzentrationswert KHb1 wird zur Korrektur der gemessenen Kurve im Bereich I, MI0, benutzt, und man gelangt für die Grundremission zu einer entsprechenden Kurve erster, besserer Näherung, GI1, indem man (in einem 4. Schritt) an der isosbestschen Wellenlänge 815 nm den bei dieser Konzentration für diese Wellenlänge durch die Hämoglobinkonzentration hervorgerufenen (zusätzlichen) Amplitudenwert von MI0 abzieht. Der sich daraus ergebende Wert wird mit Hilfe der Tabelle (Fig. 4) (oder des alternativen Verfahrensschrittes 2.2) zur Auswahl einer besser passenden Standard-Grundremissionskurve SII1 (s. Fig. 1 links) verwendet, die wiederum zur Verbesserung des Wertes der Konzentration des Hämoglobins zu einem Wert KHb2 dient. Dieser dient wiederum in der geschilderten Weise zur Ermittlung einer verbesserten Grundremissionskurve im Bereich 1, GI2. Durch mehrfaches derartiges Einsetzen kann man schließlich einen stark verbesserten Wert für die Konzentration des Hämoglobins, KHbn, erreichen.

In einem weiteren Schritt wird die Oxygenierung bestimmt, unter gleichzeitiger Verbesserung des Wertes für die Konzentration.

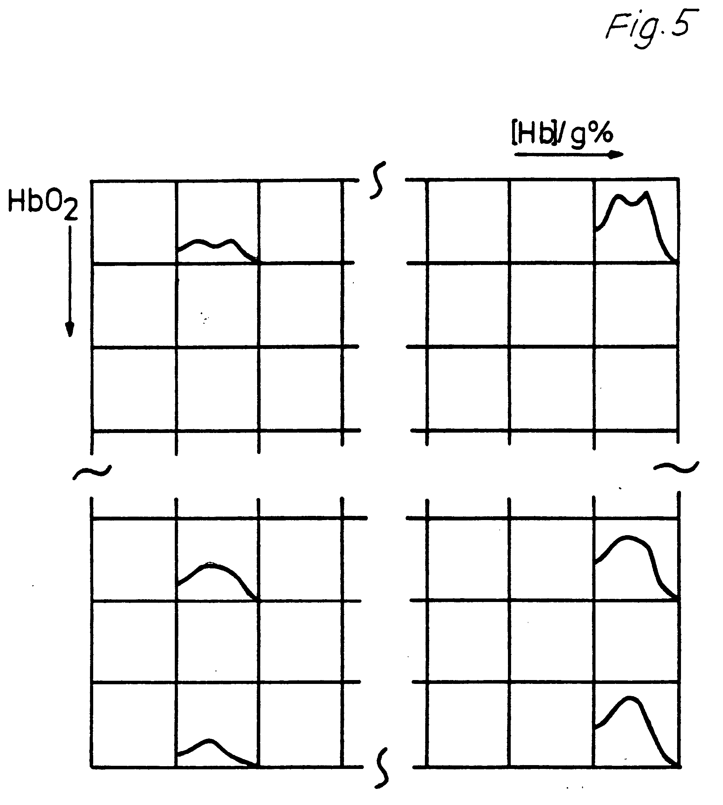

Vor Beginn der konkreten Messung wurden anhand einer sehr großen Zahl von Messungen an der gleichen Gewebeart, die mit Hämoglobin unterschiedlicher Konzentration (von 0 bis 20 %) und unterschiedlicher Oxygenierung (0 bis 100 %) perfundiert wurde,Standard-Remissionskurven mit dem gleichen Gerät, mit dem die konkrete Messung durchgeführt wird, ermittelt. Die Standard-Remissionskurven, die ihrerseits für je eine bestimmte Konzentration und Oxygenierung aus der großen Zahl von Messungen gemittelt sind, sind in einer Tabelle oder Matrix angeordnet, derart, daß z. B. die Zeilen die verschiedenen Konzentrationen bei gleicher Oxygenierung und die Spalten die verschiedenen Oxygenierungen bei gleicher Konzentration enthalten. Dies ist schematisch in Fig. 5 dargestellt.

Die gemessene Kurve MII0 wird nun mit den Kurven in der Tabelle verglichen. Dabei werden bspw. und bevorzugt die der ersten Näherung der Hämoglobinkonzentration entsprechende Spalte und je eine oder im Ausführungsbeispiel je zwei Nachbarspalten durchsucht, zu sämtlichen Werten der Oxygenierung.

Zum Vergleich der Kurven kann in einer Ausführung des Verfahrens das Integral der Fläche unter den Standard-Remissionskurven mit dem Integral unter der gemessenen Kurve MII0 verglichen werden.

Alternativ werden die Kurven nach der Methode der kleinsten Quadrate verglichen.

Das Feld der Matrix mit der optimal passenden Kurve, ergibt einen Konzentrationswert des Hämoglobins zweiter Näherung, KHb2, und einen Oxygenierungsgrad des Hämoglobins, erster Näherung, KHbO₂1.

Diese Werte werden nun bspw. wiederum verwandt, um verbesserte Werte für die Grundremissionskurve und die Konzentration zu gewinnen.

Auch hier ist ersichtlich, daß das Verfahren fortgesetzt werden kann, bis eine in Bezug auf die realistische Meßgenauigkeit hinreichende Konvergenz erreicht wird.

Die Oxygenierung des Hämoglobins läßt gleichzeitig auf die Oxydation der Zytochrome schließen. Ferner erlaubt die Grundremissionskurve nach Abzug des Hämoglobineinflusses eine genauere Bestimmung anderer Parameter im Gewebe.

Bei sehr niedriger Oxygenierung, d. h. Sauerstoff-Partialdrucken < 667 Pa (5 Torr), die im Gewebe gemessen werden, müssen andere Grundremissionskurven zugrundegelegt werden, was aber am grundsätzlichen Verfahren nichts ändert.

Fig. 2 zeigt in vergrößertem Maßstab der Kurve MI0 entsprechende gemessene Kurven aus dem Wellenlängenbereich I, einmal bei hoher Oxygenierung des Hämoglobins (M'I0ₕ) und einmal bei niedriger Oxygenierung des Hämoglobins (M'I0ₙ).

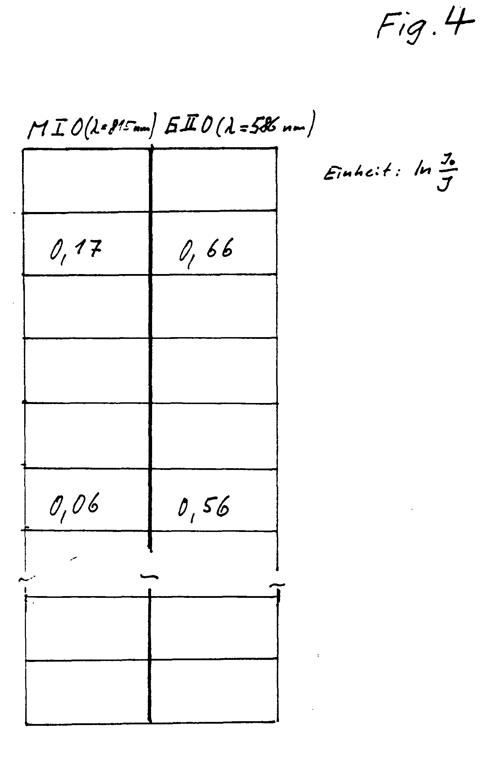

Fig. 3 zeigt in vergrößertem Maßstab den Kurven SII entsprechende Standard-Grundremissionskurven, einmal bei hoher (SIIₕ) und einmal bei niedriger SIIₙ) Oxydation der Atmungsfermente.

Genauere Angaben über eine "Konzentration" lassen sich nur unter Berücksichtigung des durch die Messung erfaßten (Mikro-) Volumens des Gewebes machen.

Das erfaßte Volumen hängt von folgenden Parametern ab:
1. der jeweils verwendeten Wellenlänge und ganz allgemein den Eigenschaften der verwendeten Lichtquelle (Leuchtfelddichte, Intensität, Stabilität)
2. den Übertragungseigenschaften des in das Gewebe einstrahlenden Lichtleiters (Akzeptanzwinkel a, Länge L, Durchmesser d, Material)
3. der Streucharakteristik des Gewebes und dem Absorptionsverhalten des bzw. der Farbstoff(e)(s)
4. den Übertragungseigenschaften des das rückgestreute Licht aufnehmenden (detektierenden) Lichtleiters (Akzeptanzwinkel a, Länge L, Durchmesser d, Material) und des nachfolgenden Detektionssystems
5. der Empfindlichkeit des lichtmessenden Systems, bspw. des Photomultipliers.

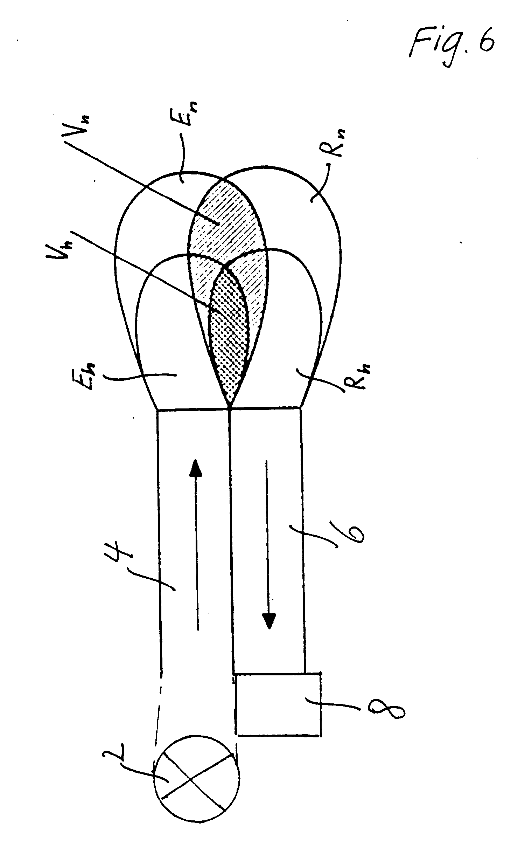

Fig. 6 zeigt schematisch die beleuchtete Lampe 2, den illuminierenden Lichtleiter 4, den detektierenden Lichtleiter 6 und den Photovervielfacher 8. Das Volumen, in den der beleuchtende Lichtleiter 4 im Gewebe einstrahlt, ist für eine hohe Hb-Konzentration mit Eh und für eine niedrige mit En angedeutet; das Volumen, aus dem, unter Berücksichtigung der Empfindlichkeit des Photovervielfachers 8, der detektierende Lichtleiter 6 Licht empfangen kann, ist mit Rₕ bzw. Rₙ bezeichnet. Das Schnittvolumen, Vₕ bzw. Vₙ, ist das der Konzentrationsmessung zugrundeliegende Volumen. Durch hohe Lichtdichte wird eine quasi diffuse Beleuchtung erreicht.

Es wird davon ausgegangen, daß das zur Erstellung der benutzten Tabellen (Matrizen) verwendete Gerät das selbe wie das zur konkreten Messung verwendete ist, so daß insofern keine Änderung des Volumens eintritt, oder daß nur Faktoren geändert werden, die herausgerechnet werden können.

Informationen über das unter bestimmten Verhältnissen erfaßte Volumen sind ebenfalls in Tabellenform erzeugbar. Die entsprechenden Werte können durch Messungen an Schnitten des Gewebes oder in Streukammern unter Simulation des Gewebes gewonnen werden.

Den oben erwähnten Konzentrationswerten werden Volumina zugrundegelegt, die sich aus den Tabellen als Erfahrungswerte ergeben. Mit Hilfe eines besonderen Verfahrens lassen sich jedoch auch die Volumina korrigieren.

Erfindungsgemäß wird als Maß für die Eindringtiefe und damit das erfaßte Volumen die Verzerrung der gemessenen Hämoglobinkurve MII0 gegenüber der aus der Matrix (Tabelle) 2 (s. Fig. 5) ermittelten Standard-Remissionskurve RSII1 verwendet. Dazu wird die gemessene Hämoglobinkurve MII0 auf die ermittelte Standardkurve an der isosbestschen Wellenlänge normiert und die Differenz wellenlängenabhängig aufgetragen.

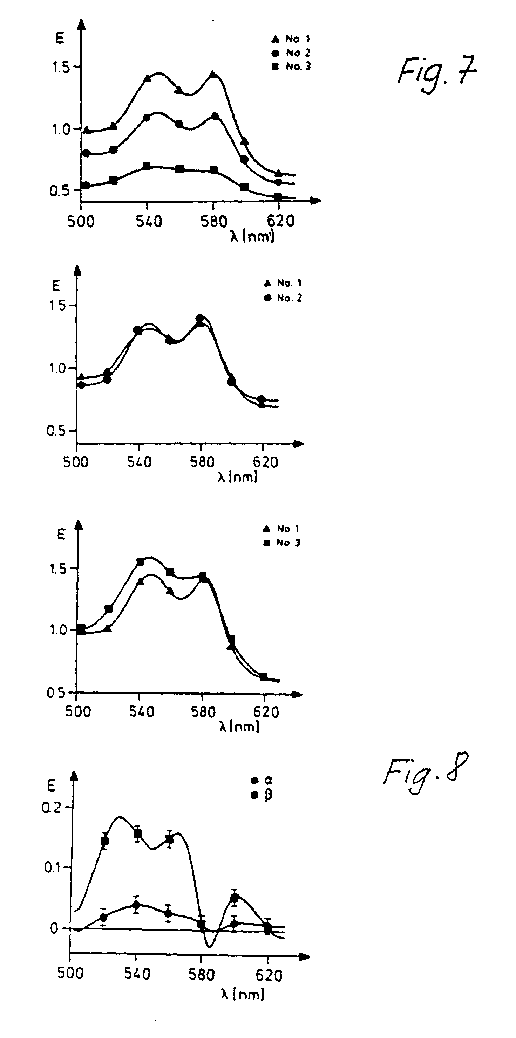

Fig. 7 zeigt (schematisch) ein Beispiel einer möglichen Standard-Remissionskurve RSII1 mit zwei Beispielen für mögliche Meßkurven MII0 und Fig. 8 die sich daraus jeweils ergebenden Differenz- oder Verzerrungskurven.

Wiederum in entsprechenden Vor-Messungen mit dem gleichen Gerät wurde eine Matrix solcher Verzerrungskurven erstellt und jedes Feld der Matrix einem erfaßten Volumen V (s. Fig. 6) zugeordnet. Diese Matrix ist hier nicht gezeigt.

Auf diese Weise läßt sich das Volumen bestimmen und wiederum als Korrekturfaktor für die bisher ermittelten Werte der Konzentration und der Oxygenierung verwenden.

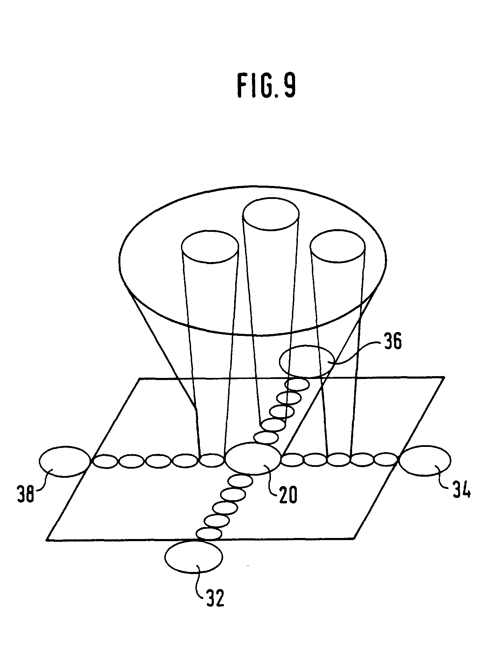

Bevorzugt wird ein zentraler beleuchtender Lichtleiter 20 und ein Feld von bspw. 10 x 10 empfangenden Lichtleitern vorgesehen (vgl. auch Fig. 9). Dies ermöglicht die Messung ganzer Topograhien von Oxygenierungs- und Farbstoffverteilungen.

Die aus den jeweiligen einzelnen Lichtleitern kommenden Informationen werden zunächst einzeln ausgewertet, wie oben für einen empfangenden Lichtleiter im Einzelnen geschildert und die Ergebnisse ergeben dann ein Topogramm der HB-Konzentration, ein Topogramm der H_{b}O₂-Konzentration und Grundremissionstopogramme.

Die Abfrage der Lichtleiter kann gleichzeitig sein, was dann eine entsprechende Anzahl der unten geschilderten Auswerteinheiten nötig macht. Bei der Kürze der Aufnahme eines vollständigen Spektrums in beiden Wellenlängenbereichen (ca. 1/100 s), können die Lichtleiter aber auch aufeinanderfolgend abgefragt werden, was einen Zeitunterschied von ca einer Sekunde ergibt, der häufig in Kauf genommen werden kann.

Der zeitliche Verlauf im Zusammenhang mit dem winkelabhängigen, korrigierten räumlichen Remissionsdiagramm, das durch die Anordnung erhalten wird, erlaubt wieder einen Rückschluß auf die Änderung der Teilchengröße.

Die Auswertung der Winkelabhängigkeit sowohl was den Abstand vom beleuchtenden Lichtleiter als auch den Umfang von Kreisen um den beleuchtenden Lichtleiter betrifft, kann zur Feststellung von räumlichen Asymmetrien genutzt werden.

Wie konkret in Fig. 9 dargestellt ist, können sich auch noch an den Seitenrändern, in deren Mitte, beleuchtende Lichtleiter 32 - 38 befinden, wodurch weitere Informationen über das Streuverhalten im Gewebe erhalten werden können. Das Einstrahlvolumen des zentralen Lichtleiters sowie die von den jeweiligen detektierenden Lichtleitern erfaßbaren Volumina werden an Einzelbeispielen in der Zeichnung schematisch dargestellt.

In Fig. 10 ist der Grundaufbau des in Erlangen entwickelten Mikrolichtleiter-Spektralphotometers gezeigt. Das Licht einer Xenon-Hochdrucklampe 40 (z. B. XBO 75 W/2, Osram) die von einer Versorgungseinrichtung 42 (stabilisiertes Netzgerät) gespeist wird, wird über ein optisches System 44 in den beleuchtenden Lichtleiter 4 eingestrahlt. Dieser ist mit dem detektierenden Lichtleiter 6 so zusammengefaßt, daß die jeweiligen Endflächen in einer Ebene und unmittelbar nebeneinander liegen (in Fig. 10 nicht gezeigt). Das Lichtleiterpaar (bzw. die Anordnung aus Fig. 9) wird dann auf die Gewebeoberfläche 46 aufgesetzt. Über den aufnehmenden Lichtleiter 6 gelangt das Licht an eine Interferenzverlaufsfilterscheibe 48. Diese enthält in der Erfindung, im Gegensatz zu den bisher für die Gewebespektrophotometrie verwendeten Interferenzverlaufsfilterscheiben, den Wellenlängenbereich von 500 bis hinauf zu 850 nm. Es ist ersichtlich, daß dann die Messung der Kurven MI0 und MII0 praktisch gleichzeitig erfolgen kann und die Reihenfolge nur von der Drehrichtung der Filterscheibe abhängt. Das Licht des von der Interferenzverlaufsfilterscheibe 48 gerade durchgelassenen Wellenlängenbereichs (die Auflösung beträgt ca. 2 nm) wird über einen Lichtleiter 50 an einen Photovervielfacher 52 gegeben. Über ein Verstärkersystem 54 gelangt das Signal zu einem Analog/Digital-Wandler 56 und nach der Digitalisierung zu einem Rechner (auch in 56) zur weiteren Verarbeitung.

Um welche Wellenlänge es sich jeweils handelt, wird dadurch ermittelt, daß an der Welle des die Filterscheibe 48 antreibenden Motors 58 eine Dekodierscheibe 60 angeordnet ist, und die Steuersignale an einen EPROM 62 abgibt. Der EPROM 62 wandelt die Steuersignale in Triggersignale für die Digitalisierung des Meßsignals durch den A/D-Wandler 56. Ferner erzeugt die Dekodierscheibe noch einen Impuls, der den Beginn jeder Scheibendrehung markiert und welcher die Digitalisierung des A/D-Wandlers initialisiert.

Das vorliegende Verfahren arbeitet mit Absolutwerten der Remission. Daher wird der Eichung der gesamten Anordnung besondere Aufmerksamkeit geschenkt.

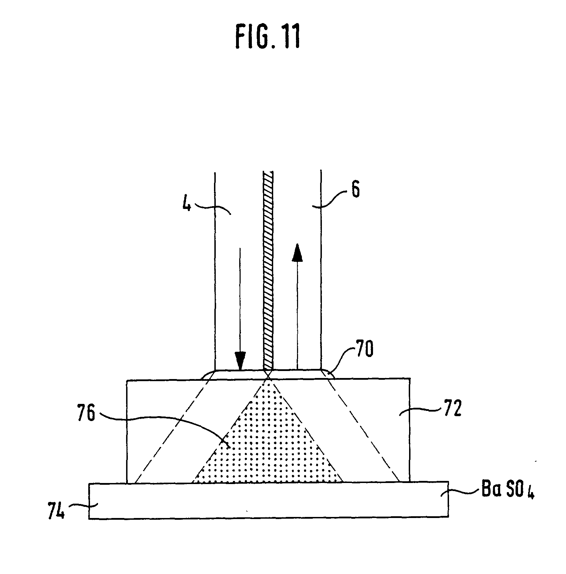

Fig. 11 zeigt die Einrichtung zur Festlegung eines weißen Spektrums. Die spektrale Verteilung des Lichtes der Xenon-Bogenlampe, die Übertragungseigenschaften der optischen Elemente (Linsen, Lichtleiter) und die spektrale Empfindlichkeit des Photovervielfachers ergeben eine wellenlängenabhängige Antwortfunktion für weißes Licht. Diese kann durch das Spektrum eines Weißstandards, hier BaSO₄, unter Verwendung der in Fig. 11 gezeigten, an sich bekannten Vorrichtung gemessen werden.

Der beleuchtende (4) und der aufnehmende (6) Lichtleiter werden in der auch bei der Messung verwendeten Anordnung in einen Tropfen Immersionsflüssigkeit 70 (0,9 % NaCI) auf eine Glasplatte 72 senkrecht aufgesetzt. Die Glasplatte schafft einen festen Abstand zum Weißnormal 74. Der Schnittbereich 76 der Lichtkegel entspricht dem Volumen V. Um den Intensitätsbereich festzulegen, muß eine wellenlängenabhängige Dunkelkurve abgespeichert werden.

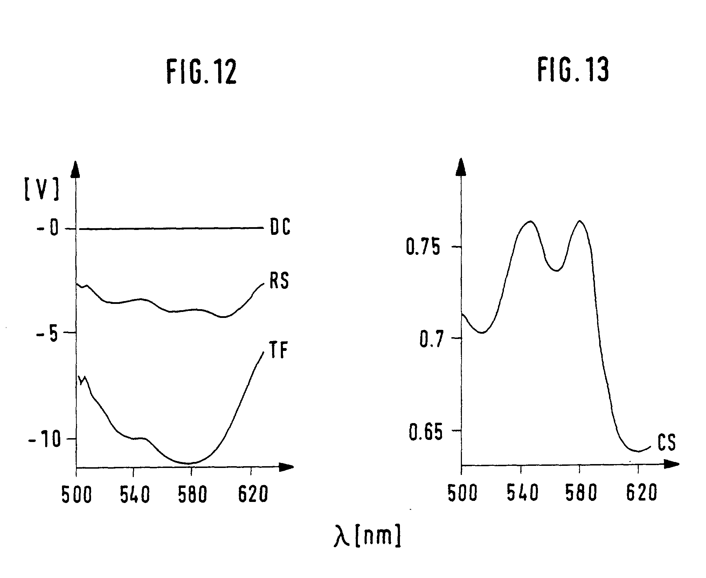

Die Korrektur der spektrophotometrischen Messungen wird in vier Schritten durchgeführt. Hierzu wird auf Fig. 12 und 13 Bezug genommen.
1. Das aufgezeichnete, gemessene Spektrum RS wird von der Dunkelkurve DC abgezogen.
2. Das Spektrum des BaSo₄-Weißnormals (BaSt) wird ebenfalls von der Dunkelkurve (DC) abgezogen (DC-BaSt = TF).
3. Die Division (DC-RS)/DC-BaSt) ergibt das korrigierte Spektrum CS-(Fig. 13).
4. Zur Darstellung wird das korrigierte Spektrum mit -1 multipliziert.

Zur Gewinnung von Absolutwerten der Konzentration, und da die Messungen der Kurven MI0 und MII0 einerseits und die Messung der Standard-Grundremissionskurven und anderer der geschilderten Vergleichskurven andererseits durchaus zu verschiedenen Zeiten durchgeführt werden kann, ist eine Absoluteichung bspw. des Photovervielfachers und anderer ggfs. verwendeter Lichtmeßeinrichtungen besonders wichtig. Hierzu wird eine Normallichtquelle, bevorzugt in Form eines Beta-Lichts, verwendet, bei welcher Zinksulfid oder ein anderer radiolumineszierender Stoff durch radioaktive Zerfallsprodukte, insbesondere Beta-Strahlen des Tritiums, angeregt wird. Dies ist näher in der älteren deutschen Patentanmeldung DE-A-3 816 489 ("Normallichtquelle") des gleichen Anmelders und der gleichen Erfinder beschrieben.

## Patentansprüche

1. Verfahren zur Bestimmung von lokalen Hämoglobin Konzentrationen in tierischen und menschlichen Geweben, bei dem Licht unterschiedlicher Wellenlängen in einen Teilbereich des Gewebes eingestrahlt wird, zumindest ein Teil des rückgestreuten Lichtes aufgefangen und die Remission in Abhängigkeit von der Wellenlänge bestimmt wird und die Konzentration von Hämoglobin aus dem spektralen Remissionsgrad bestimmt wird,
wobei in einem Schritt Strahlung aus einem ersten Wellenlängenbereich (I) in dem der Einfluß des Hämoglobins (Hb) auf die Remission gering ist, eingestrahlt und die Remission (MI0) im ersten Wellenlängenbereich (I) bestimmt wird und
wobei in einem weiteren Schritt Licht aus einem zweiten Wellenlängenbereich (II), in dem die Remission vom Einfluß des Hämoglobins dominiert wird, in den gleichen Teilbereich des Gewebes eingestrahlt wird und die Remission (MII0) im zweiten Wellenlängenbereich (II) bestimmt wird und
wobei die Daten über die Remission abgespeichert werden,
**dadurch gekennzeichnet,**
**daß** vorab eine Schar gewebeartspezifischer Standard-Grundremissionskurven an hämoglobinfreien Gewebeproben der gleichen Gewebeart über den ersten (I) und zweiten Wellenlängenbereich (II) gewonnen wird,
**daß** weiterhin die im ersten Wellenlängenbereich gemessene Remissionskurve (MI0) am zu untersuchenden Gewebe der gleichen Gewebeart dem nächstpassenden Ast im ersten Wellenlängenbereich (I) aus der Schar der Standard-Grundremissionskurven zugeordnet wird und der zugehörige Ast dieser Standard-Grundremissionskurve im zweiten Wellenlängenbereich (II) als gewebepersonenspezifische Standard-Grundremissionskurve (SII0) ausgewählt wird, wobei die Zuordnung derart erfolgt, daß die Standard-Grundremissionskurve (MI0) mit dem Wert an einer vorbestimmten isosbestschen Wellenlänge im ersten Wellenlängenbereich, der gleich oder nächst dem gemessenen Remissionswert an dieser isobestschen Wellenlänge ist, ausgewählt wird und als Wert zur Ermittlung der Hämoglobinkonzentration (KHb1) der Wert der ausgewählten Standard-Grundremissionskurve (SII0) an einer vorbestimmten isosbestschen Wellenlänge im zweiten Wellenlängenbereich (II) benutzt wird, und daß zur Bestimmung der Oxygenierung des Hämoglobin vorab anhand einer Vielzahl von Messungen an der gleichen Gewebeart mit Hämoglobin unterschiedlicher Konzentration und unterschiedlicher Oxygenierung, eine zweidimensionale Schar von Vergleichskurven erstellt wird, daß dann die Vergleichskurven mit Hämoglobinkonzentrationen in der Umgebung der ermittelten Konzentration (KHbₙ) über den ganzen Oxygenierungsbereich durchsucht werden, und die bestpassende der Vergleichskurven bei einem angenommenen Wert für die Oxygenierung einen verbesserten Wert (KHbₙ₊₁) für die Konzentration liefert.

2. Verfahren nach Anspruch 1,
**dadurch gekennzeichnet,**
**daß** anhand des für die Hämoglobinkonzentration gewonnenen Wertes (KHb1) die gemessene Kurve (MI0) im ersten Wellenlängenbereich (I) korrigiert wird, wodurch eine weitere verbesserte Näherung für die gewebepersonenspezifischen Grundremission im ersten Wellenlängenbereich (GI1) gewonen wird.

3. Verfahren nach Anspruch 2,
**dadurch gekennzeichnet,**
**daß** mit der verbesserten Kurve (GI1) anstelle der gemessenen Remissionskurve (MI0) die Schritte nach Anspruch 1 nochmals durchgeführt werden, wodurch ein besserer Näherungswert für die Hämoglobinkonzentration (KHb2) und eine weiter verbesserte Kurve (GI2) als gewebepersonenspezifische Grundremission im ersten Wellenlängenbereich (I) erhalten werden.

4. Verfahren nach Anspruch 3,
**dadurch gekennzeichnet,**
**daß** die Schritte nach Anspruch 1 unter Zugrundelegung der jeweils verbesserten Werte bzw. Kurven, wiederholt werden.

5. Verfahren nach Anspruch 1,
**dadurch gekennzeichnet,**
**daß** die Messung im ersten Wellenbereich (I) im Bereich von 630 nm bis 1000 nm durchgeführt wird.

6. Verfahren nach Anspruch 1,
**dadurch gekennzeichnet,**
**daß** die Messung im ersten Wellenbereich (I) im Bereich von 750 nm bis 850 nm durchgeführt wird.

7. Verfahren nach Anspruch 1,
**dadurch gekennzeichnet,**
**daß** die Messung im zweiten Wellenlängenbereich (II) im Bereich von 500 nm bis 620 nm durchgeführt wird.

8. Verfahren nach Anspruch 1,
**dadurch gekennzeichnet,**
**daß** die Messung im zweiten Wellenlängenbereich (II) im Bereich von 550 nm bis 570 nm durchgeführt wird.

9. Verfahren nach Anspruch 8,
**dadurch gekennzeichnet,**
**daß** die Werte für die Konzentration (KHbₙ₊₁) und die Oxygenierung anschließend zur Gewinnung einer verbesserten gewebepersonenspezifischen Grundremissionskurve (GIₙ₊₁) verwendet werden.

## Claims

1. Method of determining local haemoglobin concentrations in animal and human tissues, in which light of different wavelengths is beamed into a portion of the tissue, at least a part of the backscattered light is collected and the reflectance is determined as a function of the wavelength, and the concentration of haemoglobin is determined from the spectral reflectance factor,
in which, in one step, radiation from a first wavelength range (I), in which the influence of the haemoglobin (Hb) on the reflectance is small, is beamed in, and the reflectance (MI0) in the first wavelength range (I) is determined and
in which, in a further step, light from a second wavelength range (II), in which the reflectance is dominated by the influence of the haemoglobin, is beamed into the same portion of the tissue and the reflectance (MII0) in the second wavelength range (II) is determined, and
in which the data regarding the reflectance are stored,
**characterized in that**,
a family of tissue-type-specific standard basic reflectance curves in respect of haemoglobin-free tissue samples of the same tissue type is obtained in advance via the first (I) and second wavelength range (II),
**in that** furthermore the reflectance curve (MI0), measured in the first wavelength range, in respect of the tissue to be examined of the same tissue type, is assigned to the closest matching branch in the first wavelength range (I) from the family of standard basic reflectance curves, and the associated branch of this standard basic reflectance curve in the second wavelength range (II) is selected as the tissue-person-specific standard basic reflectance curve (SII0), the assignment being effected in such a way that the standard basic reflectance curve (MI0) with the value at a predetermined isobestic wavelength in the first wavelength range, which is the same or close to the measured reflectance value at this isobestic wavelength, is selected and the value of the selected standard basic reflectance curve (SII0) at a predetermined isobestic wavelength in the second wavelength range (II) is used as the value for determining the haemoglobin concentration (KHb1),
and **in that**, with the aid of a plurality of measurements in respect of the same tissue type with haemoglobin of varying concentrations and varying oxygenation, a two-dimensional family of comparison curves is set up in advance for determining the oxygenation of the haemoglobin,
**in that** then the comparison curves with haemoglobin concentrations in the neighbourhood of the determined concentration (KHbₙ) are examined over the entire oxygenation range, and the best match among the comparison curves, with an assumed value for the oxygenation, provides an improved value (KHbₙ₊₁) for the concentration.

2. Method according to Claim 1,
**characterized in that**,
with the aid of the value (KHb1) obtained for the haemoglobin concentration, the measured curve (MI0) in the first wavelength range (I) is corrected, as a result of which a further improved approximation for the tissue-person-specific basic reflectance in the first wavelength range (GI1) is obtained.

3. Method according to Claim 2,
**characterized in that**
the steps according to Claim 1 are carried out again with the improved curve (GI1) instead of the measured reflectance curve (MI0), as a result of which an improved approximate value for the haemoglobin concentration (KHb2) and a further improved curve (GI2) are obtained as tissue-person-specific basic reflectance in the first wavelength range (I).

4. Method according to Claim 3,
**characterized in that**
the steps according to Claim 1 are repeated on the basis of the respective improved values or curves.

5. Method according to Claim 1,
**characterized in that**
the measurement in the first wavelength range (I) is carried out in the range of 630 nm to 1000 nm.

6. Method according to Claim 1,
**characterized in that**
the measurement in the first wavelength range (I) is carried out in the range of 750 nm to 850 nm.

7. Method according to Claim 1,
**characterized in that**
the measurement in the second wavelength range (II) is carried out in the range of 500 nm to 620 nm.

8. Method according to Claim 1,
**characterized in that**
the measurement in the second wavelength range (II) is carried out in the range of 550 nm to 570 nm.

9. Method according to Claim 8,
**characterized in that**
the values for the concentration (KHbₙ₊₁) and the oxygenation are subsequently used for obtaining an improved tissue-person-specific basic reflectance curve (GIₙ₊₁).

## Revendications

1. Procédé pour déterminer des concentrations locales d'hémoglobine dans des tissus animaux et humains, dans lequel de la lumière de longueurs d'onde différentes est émise dans une zone partielle du tissu, au moins une partie de la lumière rétrodiffusée est captée et la réflexion spectrale est déterminée en fonction de la longueur d'onde, et la concentration d'hémoglobine est déterminée à partir du degré de réflexion spectrale, dans lequel au cours d'une étape un rayonnement, d'une première gamme de longueurs d'onde (I) dans laquelle l'influence de l'hémoglobine (Hb) sur la réflexion spectrale est faible, est émis et la réflexion spectrale (MIO) est déterminée dans la première gamme de longueurs d'onde (I) et dans lequel, au cours d'une autre étape, de la lumière d'une deuxième gamme de longueurs d'onde (II), dans laquelle la réflexion spectrale est dominée par l'influence de l'hémoglobine, est émise dans la même zone partielle du tissu et la réflexion spectrale (MIIO) dans la deuxième gamme de longueurs d'onde (II) est déterminée, les données concernant la réflexion spectrale étant mémorisées,
**caractérisé en ce qu'**au préalable un ensemble de courbes de réflexion spectrale de base standard, spécifiques du type de tissu, a été obtenu sur des échantillons de tissu exempts d'hémoglobine du même type de tissu, pour la première gamme de longueurs d'onde (I) et la deuxième gamme de longueurs d'onde (II), **en ce qu'**en outre, la courbe de réflexion spectrale (MIO), mesurée dans la première gamme de longueurs d'onde sur le tissu à analyser du même type de tissu, est affectée à la branche la plus adaptée dans la première gamme de longueurs d'onde (I) de l'ensemble des courbes de réflexion spectrale de base standard, et la branche correspondante de cette courbe de réflexion spectrale de base standard dans la deuxième gamme de longueurs d'onde (II) est choisie en tant que courbe de réflexion spectrale de base standard (SIIO) spécifique du tissu personnel, l'affectation s'effectuant de manière que la courbe de réflexion spectrale de base standard (MIO) soit choisie avec la valeur pour une longueur d'onde isosbest prédéterminée dans la première gamme de longueurs d'onde, qui est égale ou proche de la valeur de réflexion spectrale mesurée pour cette longueur d'onde isosbest et comme valeur pour la détermination de la concentration d'hémoglobine (KHb1) étant utilisée la valeur de courbe de réflexion spectrale de base standard (SIIO) choisie pour une longueur d'onde isosbest prédéterminée dans la deuxième gamme de longueurs d'onde (II), et **en ce que** pour la détermination de l'oxygénation de l'hémoglobine, il a été établi au préalable, à l'aide d'un grand nombre de mesures sur le même type de tissu avec de l'hémoglobine de concentration différente et d'oxygénation différente, un ensemble bidimensionnel de courbes comparatives, **en ce qu'**ensuite les courbes comparatives ont été examinées avec des concentrations d'hémoglobine proches de la concentration déterminée (KHbₙ) sur tout le domaine d'oxygénation, et la mieux adaptée des courbes comparatives fournit une valeur améliorée (KHbₙ₊₁) pour la concentration, avec une valeur supposée pour l'oxygénation.

2. Procédé selon la revendication 1, **caractérisé en ce qu'**à l'aide de la valeur (KHbl) obtenue pour la concentration d'hémoglobine, on corrige la courbe mesurée (MIO) dans la première gamme de longueurs d'onde (I), ce qui fait que l'on obtient une approximation encore améliorée pour la réflexion spectrale de base spécifique du tissu personnel dans la première gamme de longueurs d'onde (GI1).

3. Procédé selon la revendication 2, **caractérisé en ce qu'**on procède encore une fois aux étapes selon la revendication 1, avec la courbe améliorée (GI1) au lieu de la courbe de réflexion spectrale (MI0), ce qui fait que l'on obtient une meilleure valeur d'approxrmation pour la concentration d'hémoglobine (KHb2) et une courbe encore améliorée (GI2) en tant que réflexion spectrale de base spécifique du tissu personnel dans la première gamme de longeurs d'onde (I).

4. Procédé selon la revendication 3, **caractérisé en ce que** les étapes selon la revendication 1 sont renouvelées en prenant pour base les valeurs ou courbes améliorées dans chaque cas.

5. Procédé selon la revendication 1, **caractérisé en ce que** la mesure dans la première gamme d'ondes (I) est effectuée dans le domaine allant de 630 nm à 1 000 nm.

6. Procédé selon la revendication 1, **caractérisé en ce que** la mesure dans la deuxième gamme d'ondes (I) est effectuée dans le domaine allant de 750 nm à 850 nm.

7. Procédé selon la revendication 1, **caractérisé en ce que** la mesure dans la deuxième gamme de longueurs d'onde (II) est effectuée dans le domaine allant de 500 nm à 620 nm.

8. Procédé selon la revendication 1, **caractérisé en ce que** la mesure dans la deuxième gamme de longueurs d'onde (II) est effectuée dans le domaine allant de 550 nm à 570 nm.

9. Procédé selon la revendication 8, **caractérisé en ce que** les valeurs pour la concentration (KHbₙ₊₁) et l'oxygénation sont utilisées ensuite pour obtenir une courbe de réflexion spectrale de base (GIₙ₊₁) spécifique du tissu personnel.
